# EUROPEAN PATENT APPLICATION

(11) **EP 2 085 776 A1**
(43) Date of publication of application: **05.08.2009**
(21) Application number: 09151160.0
(22) Date of filing: 22.01.2009
(51) Int. Cl.: G01N 33/28, G01N 11/00, G01N 33/30, G05B 23/00

(54) **Apparatus, system, and method for onboard degraded and deadlined mechanical system alerting**

(30) Priority: 30.01.2008 US 22859
(71) Applicant: Honeywell International Inc., Morristown, NJ 07962 (US)
(72) Inventor: Wright, George L., Corrales, NM 87048 (US); Wright, Mark A., Albuquerque, NM 87111 (US)
(74) Representative: Buckley, Guy Julian

(57) **Abstract**

An apparatus, method and system are provided for determining a mechanical-system-(MS)-health measurement and a MS-health state of an MS. The MS is a system that uses a fluid that can be measured by a sensor for purposes such as lubrication or cooling. The MS-health measurement is determined by processing MS-measurement data. The MS-measurement data includes an fluid-viscosity measurement value. The fluid-viscosity measurement value is transmitted using a wired or wireless sensor interface. The MS-health state is determined by comparing the MS-health measurement to one or more MS-health thresholds. The MS-health state is either in a normal state, a degraded state, or a critical state. The MS-health state is indicated, preferably using a onboard stop-light display, on a MS-health indicator. Depending on the MS-health state, one or more MS-performance characteristics may be reduced.

## Description

### BACKGROUND OF THE INVENTION

### 1. Field of the Invention

This invention relates to the performance monitoring of a mechanical system (MS). Most particularly, this invention relates to an apparatus, method, and system for indicating a MS-health state of a MS based on MS-measurement data.

### 2. Description of Related Art

Many mechanical systems (MSs) use fluids as a lubricant, as a coolant, or for other purposes. Examples of such mechanical systems that use a fluid, such as oil, are transmissions, transfer cases, turbines, wind generators, differentials, and engines.

Typically, the ability of a MS to perform varies over time. Owners and operators of the MS commonly take various measurements of the MS to determine the MS performance. The various MS-measurements include measurements of fuel, temperature, coolant, air flow, and oil. The owner or operator of the MS typically receives specific notifications about the MS measurements, such as a "low coolant" lamp indicating a need for coolant.

Frequently, one or more MS-sensors attached to the MS make the MS-measurements, providing MS-measurement data. The MS-sensors are attached to the MS and may be connected to a control unit (CU), such as an engine control unit (ECU), via a data bus and/or copper wire. The data bus provides communication pathways between the MS-sensors and the CU using one or more wires, cables, or fiber optic links. Using the data bus, the CU can both receive MS-measurement data from the sensors and send commands to MS components.

One MS-measurement is the viscosity (i.e. resistance to flow) of a fluid, such as the viscosity of engine oil in an engine. The viscosity of the engine oil may vary in relation to the temperature of the engine oil, so an oil-viscosity measurement may be adjusted based on engine oil temperature. Either an oil-viscosity sensor or an oil viscosity and oil temperature sensor (OV&OTS) may make a oil-viscosity measurement, and the OV&OTS may make a temperature-adjusted oil-viscosity measurement. The oil-viscosity sensor or the OV&OTS may transmit an oil-viscosity measurement value over a wired connection.

The oil-viscosity measurement provides insight into wear of the MS. Specifically, wear of an MS, such as an engine, may increase if fluid viscosity, such as oil viscosity of engine oil, either increases or decreases excessively over time. In particular, oil viscosity of engine oil increases as soot, agglomerates, and other solid additives are introduced to the engine oil. Engine wear potentially increases as oil viscosity of the engine oil increases, as solid additives in the engine oil may come into contact with and grind engine components. Oil viscosity decreases as fuel, water, or other liquid additives enter into the engine oil. The ability of the engine oil to lubricate and protect the engine may decrease as the oil viscosity decreases. Engine wear potentially increases as oil viscosity of the engine oil decreases, as engine components may come into contact without effective lubrication from the engine oil.

### SUMMARY

In a first principle aspect, an exemplary embodiment provides an apparatus for indicating a mechanical-system-(MS)-health state of a MS. The apparatus comprises a user interface, a sensor interface, a processor, and data storage. The sensor interface receives MS-measurement data. The MS-measurement data comprises one or more fluid-viscosity measurement values. The data storage contains machine language instructions. The machine language instructions comprise instructions executable by the processor to determine a MS-health state of the MS. The MS-health state is based, partially or wholly, on the received MS-measurement data. The instructions executable by the processor cause the user interface to indicate the MS-health state.

In a second principle aspect, an exemplary embodiment provides a method for indicating a MS-health state of a MS. In accordance with the method, MS-measurement data is obtained that indicates a temperature-adjusted fluid-viscosity measurement of a fluid in the MS. At a processor, a MS-health measurement value is calculated. The MS-health measurement value is based, partially or wholly, on the obtained MS-measurement data. The MS-health state of the MS is determined. The MS-health state is based, partially or wholly, on the MS-health measurement value. The MS-health state of the MS is indicated.

In a third principle aspect, an exemplary embodiment provides a system for indicating an health-state of a MS. The system comprises one or more MS-sensors, a MS-health indicator operable to indicate a MS-health state of the MS, and a processor. The one or more MS-sensors are operable to obtain one or more MS-measurements of the MS. The one or more measurements of the MS comprise a fluid-viscosity measurement of a fluid in the MS. The one or more MS-sensors are operable to send data of the one or more MS-measurements. The processor is operable to determine the MS-health state based, partially or wholly on the sent data. The processor is operable to send data about the MS-health state to the MS-health indicator.

These as well as other aspects and advantages will become apparent to those of ordinary skill in the art by reading the following detailed description, with reference where appropriate to the accompanying drawings. Further, it should be understood that the embodiments described in this summary and elsewhere are intended to be examples only and do not necessarily limit the scope of the invention.

### BRIEF DESCRIPTION OF THE DRAWINGS

Exemplary embodiments of the invention are described herein with reference to the drawings, in which:
Figure 1 is a block diagram of an embodiment of the invention showing a mechanical system (MS), a fluid reservoir, a fluid sensor, a MS-sensor and an MS-alert apparatus.
Figure 2A is a trend analysis diagram depicting a mechanical-system-(MS)-health measurement graph of an example MS as an amount of solid additives increase in a fluid of the example MS over time;
Figure 2B is a trend analysis diagram depicting a MS-health measurement graph of an example MS as an amount of liquid additives increase in a fluid of the example MS over time;
Figure 3 is a block diagram of the MS-alert system;
Figure 4 is a block diagram depicting additional details of the MS-alert system;
Figure 5 is a block diagram of the computing unit and an embodiment of the user interface of the MS-alert apparatus, wherein the user interface comprises a visual MS-health indicator, an input device, and an aural MS-health indicator.
Figure 6A depicts an exemplary embodiment of the user interface, wherein the visual MS-health indicator comprises a stop-light display;
Figure 6B depicts an exemplary embodiment of the user interface, wherein the visual MS-health indicator comprises a stop-light display and the input device comprises a touch screen;
Figure 6C depicts an exemplary embodiment of the user interface, wherein the visual MS-health indicator comprises a stop-light display and the input device comprises a keypad;
Figure 7 is a block diagram of the MS-alert apparatus of the MS-alert system connected to an external computing device via a network interface;
Figure 8 shows an exemplary format for a MS-health record;
Figure 9 is a trend analysis diagram indicating both the MS-health state and the MS-health measurement over time for one MS;
Figure 10 shows a fleet-health diagram with a title and three fleet-health sub-diagrams; and
Figure 11 is a flowchart describing a method for indicating a MS-health state of an engine.
Reference numerals are shown in the drawings to identify various elements of the drawings. Drawing elements having identical reference numerals are substantially identical or identical elements.

### DETAILED DESCRIPTION OF EXEMPLARY EMBODIMENTS

### 1. Overview

Many mechanical systems (MSs) use a fluid while in operation as a lubricant, as a coolant, or for other purposes. The invention described herein is generally applicable to mechanical systems that use fluid while in operation. The fluid may be a gaseous fluid, such as air, or a liquid fluid, such as water, anti-freeze (e.g., an ethylene glycol solution), or petroleum oil. Examples of such mechanical systems that use a fluid, such as oil, are transmissions, transfer cases, turbines, wind generators, differentials, and engines. By way of non-limitative example, an MS that uses a fluid while in operation is an engine using engine oil while in operation.

An embodiment of the invention is an MS-alert system that determines an overall performance measure of an engine termed the "MS-health state." To determine the MS-health state, first MS-measurement data is obtained. A MS-health measurement then is determined based, at least in part, on the obtained MS-measurement data. The MS-health state is determined by comparing the MS-health measurement to one or more MS-health thresholds. Preferably, the MS-health state is in of one of three states: normal, degraded, or critical.

The obtained MS-measurement data comprises fluid-measurement data, such as fluid-viscosity measurement data. The obtained MS-measurement data also may comprise data about one or more other MS-measurements. Other MS-measurements include, but are not limited to, fuel flow, air flow, speed, revolutions per minute (RPMs), coolant, and temperature.

The MS-health measurement is determined based, at least in part, on the obtained MS-measurement data. The MS-health measurement may be determined by selecting the oil-viscosity measurement data as the MS-health measurement or by calculating an average of the obtained MS-measurement data.

The engine alert system may alter one or more engine-performance characteristics based on the determined MS-health state. The engine alert system may alter the one or more engine-performance characteristics by requesting an engine control unit of the engine to alter the one or more engine-performance characteristics. An example of an engine-performance characteristic is the fuel-flow of the engine. The engine alert system may determine that the MS-health state is degraded or critical and responsively alter (e.g., reduce or stop) the fuel-flow of the engine. Thereafter, the engine-alert system may determine that the MS-health state is normal and responsively alter (e.g., increase) the fuel-flow of the engine.

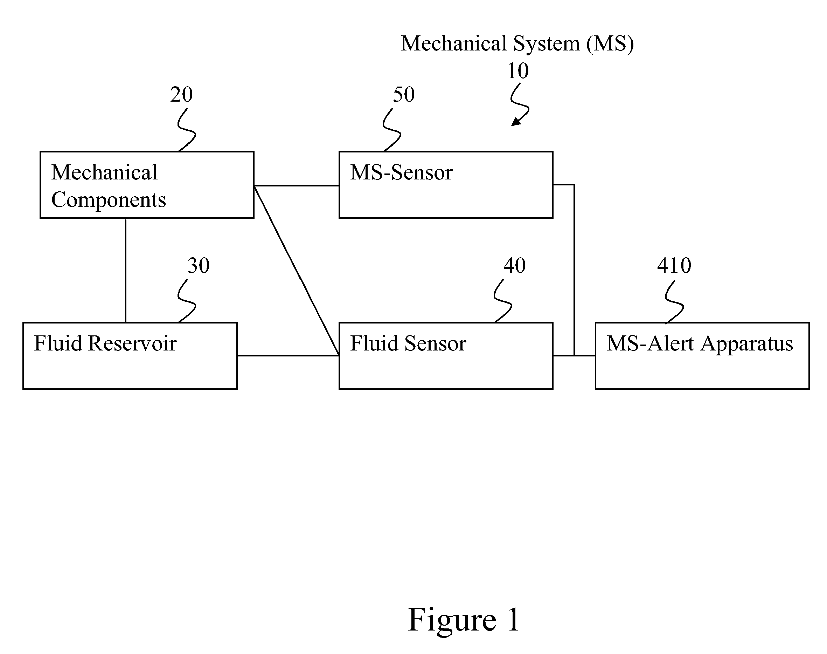

Figure 1 is a block diagram of an embodiment of the invention showing a mechanical system (MS) 10 with mechanical components 20, a fluid reservoir 30, a fluid sensor 40, a MS-sensor 50, and an MS-alert apparatus 410. A mechanical system (MS) may have mechanical components to perform one or more functions. For example, the mechanical components of a vehicle engine may comprise part or all of the drive-train, fuel, brake, exhaust, coolant, hydraulics, and other systems that enable the vehicle engine to perform the function of propelling a vehicle. The mechanical components may require use of one or more fluids to lubricate, cool, or otherwise enable the performance of the mechanical components. A fluid may be stored in a fluid reservoir. As shown in Figure 1, the MS 10 comprises the fluid reservoir 30. An example fluid reservoir is an oil drain pan for storing engine oil of a vehicle engine.

One or more fluid sensors may be connected to the mechanical components and/or the fluid reservoir of the engine to obtain one or more measurements of a fluid in the MS. As shown in Figure 1, the fluid sensor 40 is connected to both the mechanical components 20 and the fluid reservoir 30 of the MS 10. One or more MS-sensors may be connected to a MS to obtain one or more measurements of the MS, such as fuel flow, air flow, speed, RPs, coolant, and temperature. Figure 1 shows the MS-sensor 50 connected to the mechanical components 20.

An MS-alert apparatus may be connected to the MS and to one or more sensors to obtain MS-measurement data from the one or more sensors. Figure 1 shows the MS-alert apparatus 410 connected to the fluid sensor 40 and the MS-sensor 50. The MS-alert apparatus 410 may obtain MS-measurement data from the fluid sensor 40 and/or the MS-sensor 50. As described below, the MS-alert apparatus may determine a MS-health state from the obtained MS-measurement data.

### 2. The MS-health State and MS-health Measurement

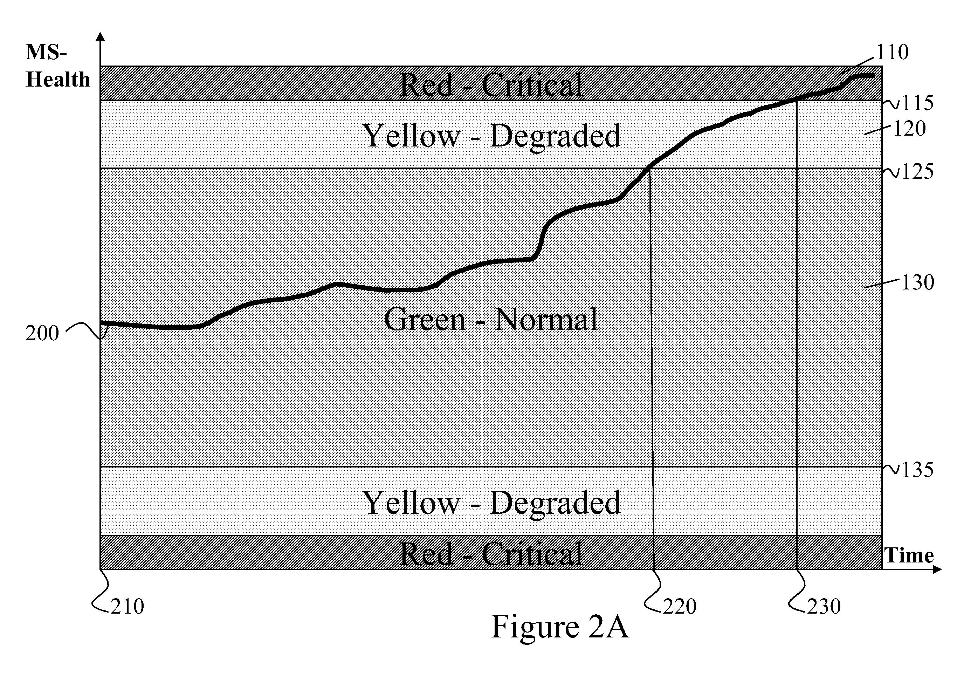

Figure 2A is a trend analysis diagram depicting a MS-health measurement graph 200 of an example MS (e.g., an engine) as an amount of solid additives increase over time in an example fluid of the MS (e.g., engine oil). The MS-health measurement graph 200 represents a plurality of MS-health measurements determined over time. Each MS-health measurement is based, at least in part, on fluid-measurement data. For the example fluid of engine oil, the fluid-measurement data may be oil-viscosity measurement data, which may comprise data from one or more measurements of oil viscosity of the engine oil in the engine. The oil-viscosity measurement data may include data on one or more temperature-adjusted oil viscosity measurements of the engine oil.

Figure 2A shows the MS-health measurement graph 200 generally increasing over time, indicating a trend of the plurality of MS-health measurements to increase over time as the amount of solid additives increase in the engine oil over time. The MS-health measurement graph 200 may reach one or more MS-health thresholds over time. Figure 1 shows the MS-health measurement graph 200 starting at time 210, reaching an upper normal MS-health threshold 125 at time 220, and then reaching an upper degraded MS-health threshold 115 at time 230.

A MS-health state may be determined using the MS-health measurement graph 200 by comparing the MS-health measurement graph 200 with the one or more MS-health thresholds. Figure 2A shows the MS-health state in a "normal" MS-health state 130 between times 210 and 220 as the MS-health measurement graph 200 is between the upper normal threshold 125 and the lower normal threshold 135 during that time span. Figure 2A also shows the MS-health state in a "degraded" MS-health state 120 between times 220 and 230 as the MS-health measurement graph 200 is between the upper normal threshold 125 and the upper degraded threshold 115 during that time span, and the MS-health state 100 in a "critical" MS-health state 110 after time 230, as the MS-health measurement graph 200 exceeds the upper degraded threshold 115 after time 230.

Colors may be associated with the MS-health states. Figure 2A associates red with the critical MS-health state 110, yellow with the degraded MS-health state 120, and green with the normal MS-health state 130.

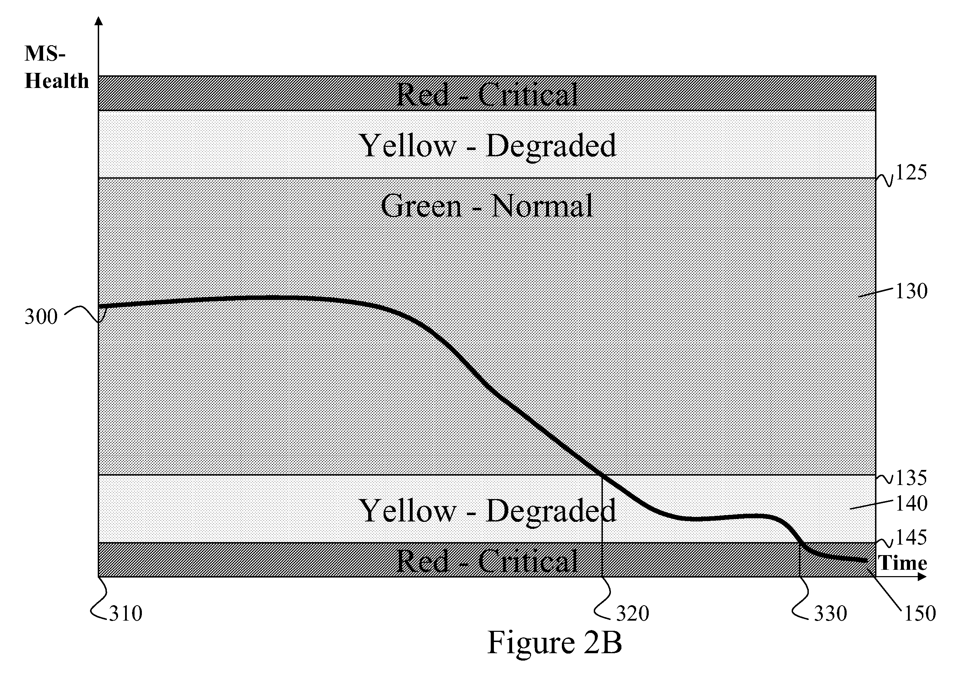

Figure 2B is a trend analysis diagram depicting a MS-health measurement graph 300 of an example MS (e.g., an engine) as an amount of solid additives decrease over time in an example fluid of the MS (e.g., engine oil). The MS-health measurement graph 300 represents a plurality of MS-health measurements determined over time. Figure 2B shows the MS-health measurement graph 300 generally decreasing over time, indicating a trend of the plurality of MS-health measurements to decrease over time as the amount of liquid additives increase in the engine oil over time. The MS-health measurement graph 300 may reach one or more MS-health thresholds over time. Figure 2B shows the MS-health measurement graph 300 starting at time 310, reaching the lower normal threshold 135 at time 320, and later reaching a lower degraded MS-health threshold 145 at time 330.

The MS-health state may be determined using the MS-health measurement graph 300. Figure 2B shows the MS-health state in a normal MS-health state 130 between times 310 and 320 as the MS-health measurement graph 300 is between the upper normal threshold 125 and the lower normal threshold 135 during that time span. Figure 2B shows the MS-health state in a degraded MS-health state 140 between times 320 and 330 as the MS-health measurement graph 300 is between the lower normal threshold 135 and the lower degraded threshold 145 during that time span, and in critical MS-health state 150 after time 330, as the MS-health measurement graph 300 is below the lower degraded threshold 145 after time 330.

### 3. The Mechanical-System-(MS)-Alert System and Apparatus

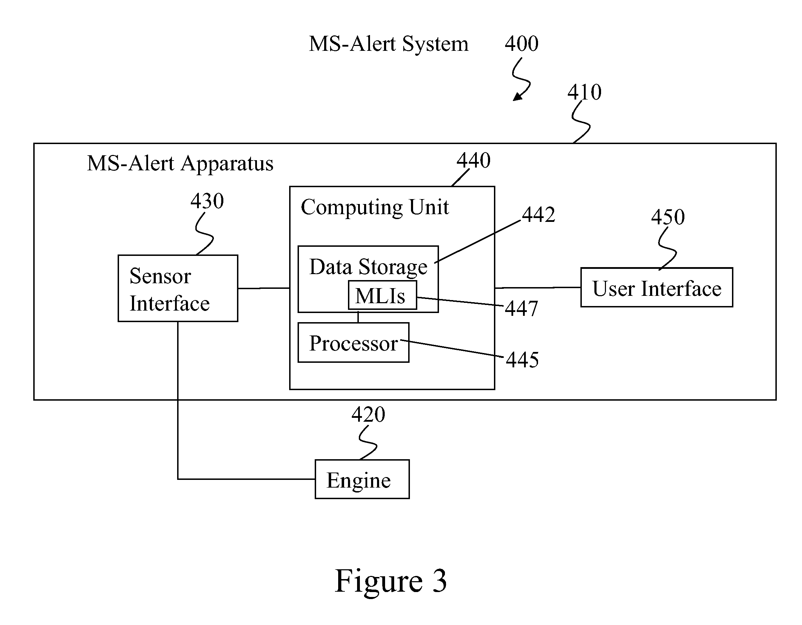

Figure 3 is a block diagram of a mechanical-system-(MS)-alert system 400. The MS-alert system 400 comprises an MS-alert apparatus 410 connected with an MS. Figure 3 shows the MS-alert apparatus 410 connected to an engine 420. The engine 420 is an example of a MS. The MS-alert apparatus 410 comprises a sensor interface 430, a computing unit 440, and a user interface 450. Both the sensor interface 430 and the user interface 450 are connected to the computing unit 440. The sensor interface 430 may send MS-measurement data, including oil-viscosity measurement data to the computing unit 440. Alternatively, the computing unit 440 may receive MS-measurement data through a direct connection to the engine 420.

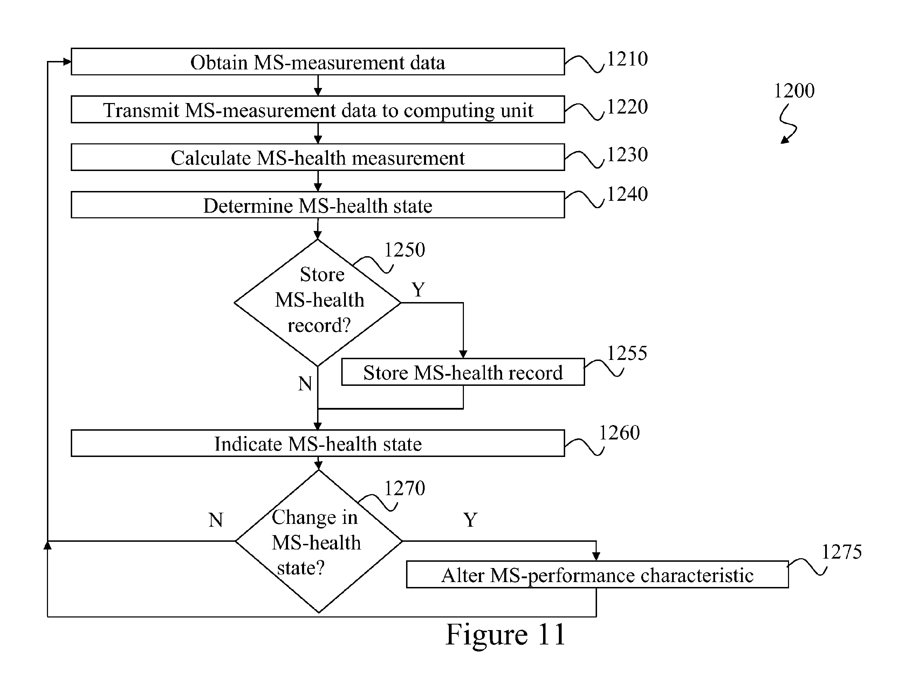

The computing unit 440 may determine a MS-health measurement value based, at least in part, on the MS-measurement data. The MS-health measurement value is determined based, at least in part, on fluid-measurement data, and preferably on oil-viscosity measurement data. The discussion below of block 1230 of Figure 11 provides an example determination of the MS-health measurement value. Once the computing unit 440 calculates the MS-health measurement value, the computing unit 440 may determine the MS-health state. Determination of the MS-health state may comprise comparing the MS-health measurement value to one or more MS-health thresholds. The discussion below of block 1240 of Figure 11 provides an example determination of the MS-health state.

The computing unit 440 may cause the user interface 450 to indicate the MS-health state. In particular, computing unit 440 may send the MS-health state to the user interface 450. The user interface 450 may comprise a display operable to indicate the MS-health state to a user of MS-alert system 400, a speaker or other device operable to produce aural notifications, and an input device.

The computing unit 440 comprises data storage 442, a processor 445, and machine language instructions (MLIs) 447. The data storage 442 has sufficient storage capacity to: (i) store MLIs 447, (ii) enable the execution of the MLIs 447 on processor 445, and (iii) contain one or more MS-health records (described below). The data storage 442 comprises one or more storage devices known or to be developed operable to store data, utilizing technologies such as read-only memory (ROM), random access memory (RAM), removable disk drive memory, hard disk memory, magnetic tape memory, and/or flash memory.

The processor 445 may include one or more central processing units, computer processors, mobile processors, digital signal processors (DSPs), microprocessors, computer chips, and similar processing units known and to be developed that execute machine language instructions and processes data. The MLIs 447 contained in the data storage 442 include instructions executable by the processor 445 to perform some or all of the functions of computing unit 440 or processor 445 described herein.

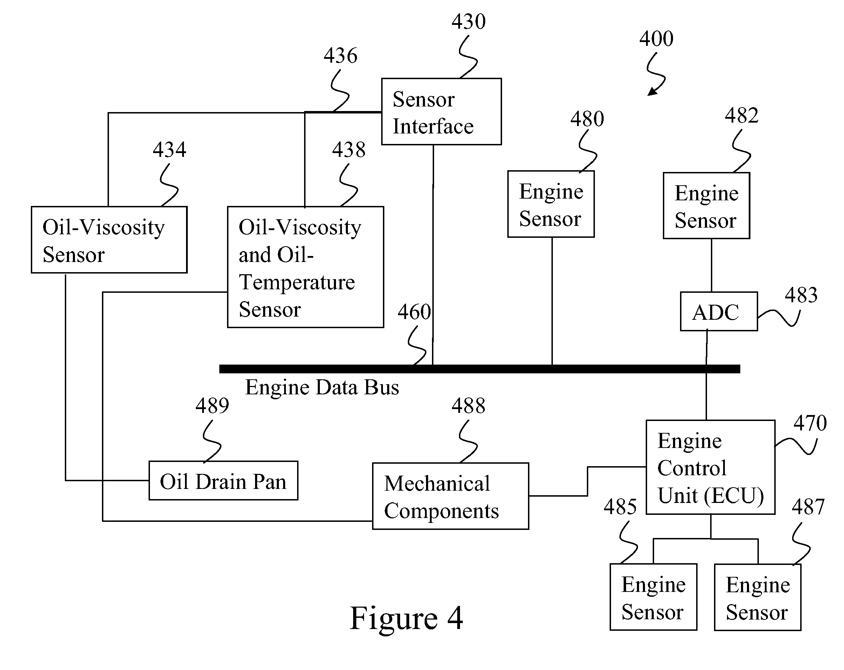

Figure 4 is a block diagram depicting additional details of the MS-alert system 400 in the context of an engine 420 as an MS. In the context of the engine 420 as the MS, engine oil is an example fluid used in the MS and oil-viscosity measurement data is an example of both MS-measurement data and fluid-measurement data.

As shown in Figure 4, the sensor interface 430 is connected to an oil viscosity sensor 434, an oil viscosity and oil temperature sensor (OV&OTS) 438, and an engine data bus 460. The engine data bus 460 is connected to an engine control unit (ECU) 470, an engine sensor 480 and an analog-to-digital converter (ADC) 483. The ADC 483 is connected to an engine sensor 482. The ECU 470 is connected to engine sensors 485 and 487 and mechanical components 488. The mechanical components 488 comprise components to perform the various functions of the engine 420, such as the drive-train, fuel, brake, exhaust, coolant, and/or hydraulics system.

The oil viscosity sensor 434, the OV&OTS 438, and the engine sensors 480, 482, 485, and 487 are operable to obtain MS-measurement data, such as oil-viscosity measurement data, and sensor interface 430 is operable to receive the obtained MS-measurement data. The OV&OTS 438 preferably obtains oil-viscosity measurement data with a Biode/Vectron bolt shear oil viscosity sensor. While Figure 4 shows the sensor interface 430 coupled with a variety of engine sensors, the sensor interface 430 may be coupled with more or fewer sensors than shown in Figure 4.

In particular, the sensor interface 430 is coupled with an oil sensor. The oil sensor is a sensor selected from the group consisting of the oil viscosity sensor 434, the OV&OTS 438, and a bolt shear viscosity sensor. The oil sensor may be installed by an original-equipment manufacturer of engine 420 or may be installed as an after-market component to engine 420, preferably before or during the installation of the components of the MS-alert apparatus 410.

The oil sensor preferably is packaged as a replacement oil drain plug that threads into a fluid reservoir of the engine 420, such as an oil drain pan 489, so as to minimize the time and difficulty of installing the oil sensor. The oil sensor may be connected to mechanical components 488 and/or the oil drain pan 489 to obtain MS-measurement data, such as oil-viscosity measurement data about engine oil in the engine 420. Figure 4 shows, as examples, the oil viscosity sensor 434 connected to oil drain pan 489 and OV&OTS 438 connected to the mechanical components 488.

Figure 4 shows the oil viscosity sensor 434 and the OV&OTS 438 connected to the sensor interface 430 via a communication link 436. The communication link 436 is operable to communicate oil-viscosity measurement data from the oil viscosity sensor 434 and/or the OV&OTS 438 to the sensor interface 430. The communication link 436 may be wired or wireless. If the communication link 436 is a wired connection, the communication link 436 may be, but is not limited to, a wire, a ribbon connector, electrical cable, coaxial cable, fiber optic cable, and/or a connection compliant to the ISO 11898 standard for controller area networks.

If the communication link 436 is a wireless connection, then preferably the wireless connection utilizes one or more wireless interface devices compliant with the Institute of Electrical and Electronics Engineers (IEEE) 802.15.4 standard. The IEEE 802.15.4 standard is specified in IEEE Computer Society, *IEEE Std. 802.15.4-2006, IEEE Standard for Information Technology - Telecommunications and Information Exchange Between Systems* - *Local and Metropolitan Area Networks - Specific Requirements - Part 15.4: Wireless Medium Access Control (MAC) and Physical Layer (PHY) Specifications for Low-Rate Wireless Personal Area Networks (WPANs)* (IEEE Sept. 8, 2006) ("ZigBee"). ZigBee wireless interface devices are intended for use in embedded applications that use relatively short range connections with low data rates and low power consumption, such as an embodiment of the instant invention. Other wireless interfaces, such as an IEEE 802.11 standard ("Wi-Fi") compliant device for longer range connections, may be used to provide the communication link 436.

Figure 4 shows the ECU 470 connected to the engine data bus 460. The ECU 470 is an example of a control unit (CU) of an MS. A control unit of an MS may place MS-measurement data and/or MS-control requests on a data bus. The MS-control requests may alter one or more MS-performance characteristics of the MS. In response to a MS-control request, an MS-control responses may be generated by the CU. In the context of ECU 470 acting as CU, the data bus is an "engine data bus", a MS-control request is termed an "engine-control request," an MS-performance characteristic is termed an "engine-performance characteristic" and a MS-control response is termed an "engine-control response." Figure 4 also shows the ECU 470 connected to mechanical components 488.

Figure 4 shows the sensor interface 430 connected to the engine data bus 460. Figure 4 also shows the engine data bus 460 connected, via the sensor interface 430, to the oil viscosity sensor 434 and the OV&OTS 438. The oil viscosity sensor 434 and/or the OV&OTS 438 may place oil-viscosity measurement data on the engine data bus 460 for use by the ECU 470. In an alternative embodiment, the oil viscosity sensor 434 and/or the OV&OTS 438 may be directly connected to engine data bus 460. In this alternative embodiment, the sensor interface 430 may receive oil-viscosity measurement data from oil viscosity sensor 434 and/or OV&OTS 438 via the engine data bus 460.

The engine sensors 480, 482, 485, and 487 may place MS-measurement data on the engine data bus 460. The engine sensors 480, 482, 485, and 487 may be connected to the engine data bus in one of several fashions. For examples, the engine sensor 480 is directly connected to the engine data bus 460, the engine sensor 482 is connected to the engine data bus 460 via ADC 483, and the engine sensors 485 and 487 are connected to the engine data bus 460 via the ECU 470. The engine sensors 480, 482, 485, and 487 may comprise sensors that measure and/or obtain data about fuel flow, engine temperature, engine coolant, air flow, engine oil, and/or other characteristics of the engine 420.

The ECU 470 may place MS-measurement data and engine-control requests on the engine data bus 460. The sensor interface 430 may be configured to read MS-measurement data placed on the engine data bus 460 by the ECU 470 and the engine sensors 480, 482, 485, and 487. The sensor interface 430 may send the MS-measurement data from the engine data bus 460 to the computing unit 440.

The MS-alert system 400 may cause the alteration of one or more engine-performance characteristics by sending one or more engine-control requests to the ECU 470. The computing unit 440 of the MS-alert system 400 may send the one or more engine-control requests to the sensor interface 430. Responsively, the sensor interface 430 may send the one or more engine-control requests to the ECU 470 via the engine data bus 460. In response to receiving the one or more engine-control requests, the ECU 470 may alter one or more engine-performance characteristics of the engine 420 and/or send one or more engine-control responses. The computing unit 440 may interpret the one or more engine-control responses from the ECU 470 and may cause the user interface 450 to display information about the one or more engine-control responses. The MS-alert system 400 may cause the alteration of the one or more engine-performance characteristics with or without input from a user of the MS-alert system 400 and may or may not inform the user about the alteration.

Altering engine-performance characteristics may include reducing engine-performance characteristics or increasing engine-performance characteristics. Examples of reducing engine-performance characteristics are: (1) limiting fuel flow and thus a maximum number of revolutions per minute (RPMs) of the engine 420, and (2) shutting down the engine by stopping the fuel flow of the engine 420. Examples of increasing engine-performance characteristics are: (1) allowing fuel to flow within the engine 420 and (2) increasing the fuel flow of the engine 420. The result of altering engine-performance characteristics may limit a speed of the engine, an amount of time to operate the engine, or a distance the engine can travel, or remove limitations on the engine speed, the operating time, or the distance.

The computing unit 440 may cause the alteration of the one or more engine-performance characteristics, based, at least in part, on the MS-health state and/or the MS-health measurement. In particular, the computing unit 440 may cause the alteration of the one or more engine-performance characteristics, based, at least in part, upon determining that the MS-health state has changed. To determine if the MS-health state has changed, the computing unit 440 first may determine a current MS-health state based, at least in part, on a current MS-health measurement as described above. Then, the computing unit 440 may compare the current MS-health state to a previous MS-health state. Preferably, the previous MS-health state is stored in the data storage 442.

For example, the computing unit 440 may determine the MS-health state is in, or has changed to, a degraded state, and subsequently send one or more engine-control requests to the ECU 470 to reduce fuel flow of the engine 420. In response to the engine-control request to reduce fuel flow, the ECU 470 may reduce the fuel flow, thereby reducing the RPMs, of the engine 420, and may provide one or more engine-control responses to the computing unit 440. The computing unit 440 may cause the user interface 450 to display information about the one or more engine-control responses. Similarly, the computing unit 440 may request the ECU 470 to further decrease and/or stop fuel flow if the MS-health state is in, or changes to, a critical state, and may cause the user interface 450 to display information about any subsequent engine-control responses from the ECU 470.

For another example, the computing unit 440 may determine that the MS-health state has changed from a critical state to the degraded state and subsequently the computing unit 440 may send one or more engine-control requests to the ECU 470 to allow and/or increase fuel flow of the engine 420. In response to the engine-control request, the ECU 470 may allow and/or increase the fuel flow, thereby increasing the RPMs of the engine 420, and may provide one or more engine-control responses to the computing unit 440. The computing unit 440 may cause the user interface 450 to display information about the one or more engine-control responses. Similarly, the computing unit 440 may request the ECU 470 to further increase fuel flow if the MS-health state is in, or changes to, a normal state, and may display information about any subsequent engine-control responses from the ECU 470.

Alternatively, some or all of the instructions of the MLIs 447 may be in the ECU 470, including, but not limited to, the functionality to cause the alteration of the one or more engine-performance characteristics. In this alternative, the ECU 470 may receive the MS-health state and/or MS-health measurement from the computing unit 440 (via the sensor interface 430 and the engine control bus 460) or the ECU 470 itself may determine the MS-health state and/or MS-health measurement. The ECU 470 then may alter the performance of the engine 420 based, at least in part, on the MS-health state and/or the MS-health measurement.

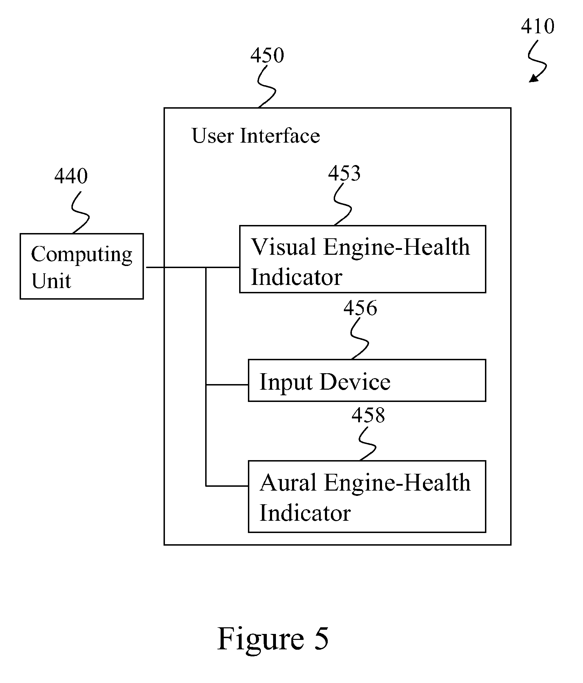

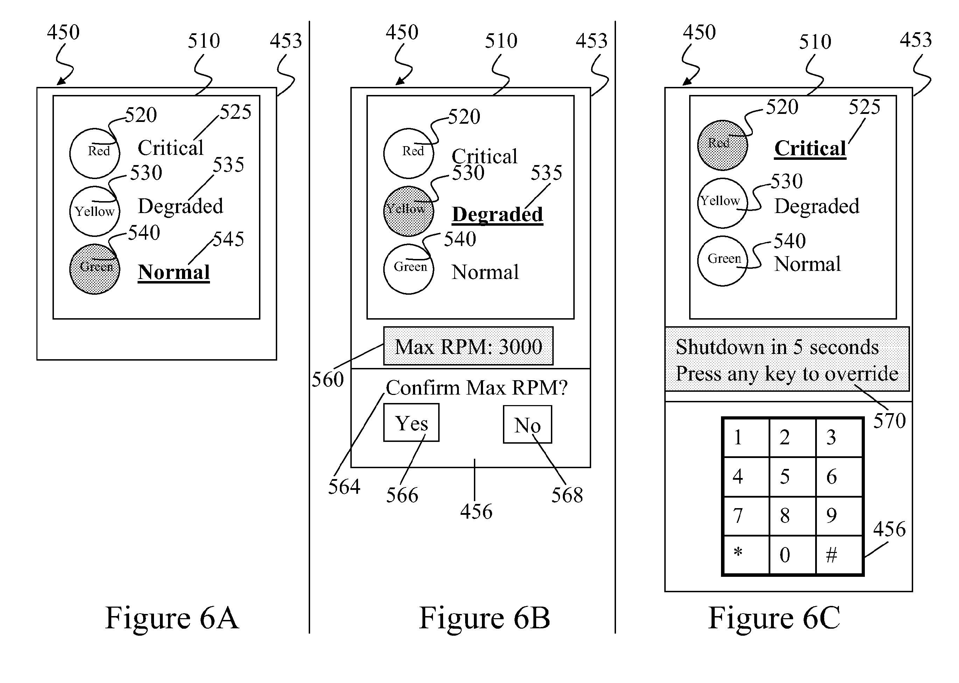

Figure 5 is a block diagram of the computing unit 440 and an embodiment of the user interface 450 of the MS-alert apparatus 410, wherein the user interface 450 comprises a visual MS-health indicator 453, an input device 456, and an aural MS-health indicator 458. The visual MS-health indicator 453 is a visual display, which is preferably a "stop-light display" as shown in Figures 6A, 6B, and 6C and described below. The visual display may also provide a textual and/or a numerical indication of the MS-health state. The visual MS-health indicator 453 may be any device, known or to be developed, operable to visually display text and/or graphical information, such as a cathode ray tube and/or a liquid crystal display.

The input device 456 may allow the user of the MS-alert system 400 to provide input to the computing unit 440. The input device 456 may be any device, known or to be developed, operable to provide user input to the computing unit 440, such as a keyboard, a key pad, a computer mouse, a remote control, a touch screen, a track ball, a joystick, and/or steering wheel controls. The computing unit 440 may use the user input to the MS-alert system 400 for user configuration, user control, and/or any other purpose that requires user input to the MS-alert system 400.

The aural MS-health indicator 458 may be a device known or to be developed capable of playing one or more aural indicators to a user of the MS-alert system 400, such as a loudspeaker. The one or more aural indicators, such as speech, beeps, bells, tones, whistles, buzzers, and musical notes, may be used to indicate a MS-health state or a change in the MS-health state. As an example, a constant aural indicator may be played to indicate a MS-health state, such as a constantly ringing bell when the MS-health state is critical. As another example, an aural indicator may be played when the MS-health state changes from normal to degraded (or vice versa) and a different aural indicator may be played when the MS-health state changes from degraded to critical (or vice versa).

Figure 6A depicts an exemplary embodiment of the user interface 450, wherein the visual MS-health indicator 453 comprises a stop-light display 510. The stop-light display 510 may comprise graphical indicators of the MS-health state. Figure 6A shows stop-light display 510 with three graphical indicators: a critical graphical indicator 520, a degraded graphical indicator 530, and a normal graphical indicator 540. Figure 6A also shows the graphical indicators 520, 530, and 540 as circular in shape and arranged with the critical graphical indicator 520 above the degraded graphical indicator 530, and the degraded graphical indicator 530 above the normal graphical indicator 540.

A stop-light display also may comprise textual indications of the MS-health state. Figure 6A shows stop-light display with three textual indicators: a critical textual indicator 525, a degraded textual indicator 535, and a normal textual indicator 545. Alternatively, the visual MS-health indicator 453 may display graphical and/or textual indicators of the MS-health display without use of a stop-light indicator. It is to be understood that the visual MS-health indicator 453 may comprise more or fewer indicators, preferably depending on a corresponding increase or decrease of possible MS-health states.

A graphical and/or a textual indicator may indicate a MS-health state. In Figures 6A, 6B, and 6C, a MS-health state is indicated by showing: (i) a graphical indicator as shaded and (ii) a textual indicator in a bold and underlined typeface. Figure 6A indicates the normal MS-health state by showing the normal graphical indicator 540 as shaded and the normal textual indicator 545 in a bold and underlined typeface. The MS-alert system 400 may change other aspects of a graphical indicator to indicate a MS-health state, such as, but not limited to, the color, brightness, size, and shape of the graphical indicator. The MS-alert system 400 may change other aspects of a textual indicator to indicate a MS-health state, such as, but not limited to foreground color, background color, brightness, size, position, and wording of the textual indicator. Further, a textual and/or graphical indicator may flash and/or change position on the visual MS-health indicator 453 to indicate a MS-health state.

Figure 6B depicts an exemplary embodiment of the user interface 450, wherein the visual MS-health indicator 453 comprises the stop-light display 510 and the input device 456 comprises a touch screen. Figure 6B indicates the degraded MS-health state by showing the degraded graphical indicator 530 as shaded and the degraded textual indicator 535 in a bold and underlined typeface.

The MS-alert system 400 may cause the user interface 450 to display one or more alert messages that describe the altered MS-performance characteristic(s). Figure 6B shows the MS-alert system 400 has caused the visual MS-health indicator 453 to display an alert message 560. The alert message 560 informs the user of the MS-alert system 400 that the MS-alert system 400 has altered one or more MS-performance characteristics of an MS, such as the engine 420, to prevent the MS from operating above a maximum of 3,000 RPMs.

The MS-alert system 400 may cause the user interface 450 to display other non-alert messages, such as user configuration messages, help messages, and information messages on the MS-health state or MS-health measurement. User configuration messages may be used to prompt and/or guide a user of the MS-alert system 400 to perform functions such as, but not limited to: (i) turning on or off the MS-alert system 400, (ii) configuring the user interface 450 to allow or inhibit aural indicators and/or graphical indicators, (iii) specifying a set of one or more key MS-values of an MS (e.g., engine 420), such as a type of engine or a type of oil, and (iv) allowing and/or inhibiting the use of engine-control requests. Help messages may inform the user how to use and operate the MS-alert system 400. Information messages may be used by MS-alert system 400 to inform the user about the MS, such as changes in MS-health, the value of the MS-health measurement, and information obtained about the MS, such as information obtained about the engine 420 via sensor interface 430.

The MS-alert system 400 may request input from the user. Figure 6B shows an input request message 564 displayed on the visual MS-health indicator 453 requesting the user to confirm the maximum RPM setting. The MS-alert system 400 may request input for user control. Figure 6B shows that the user input may be either "Yes" input 566 or "No" input 568. If the user selects "Yes" input 566, the MS-alert system 400 will limit a MS (e.g., the engine 420) to 3,000 RPMs. If the user selects "No" input 568, the MS-alert system 400 will not limit the MS to 3,000 RPMs. The MS-alert system 400 may request input for other actions, including but not limited to, user configuration of the MS-alert system 400, adding and/or removing the display of help and information messages, and confirming user input to the MS-alert system 400.

The user interface 450 may be used to determine a specified table of MS-health thresholds. The specified table of MS-health thresholds may be stored in any other data structure that can store threshold values and retrieve them based on one or more key values, such as a table (e.g. a hash table or a lookup table), a relational data base, a linked list, or a tree structure.

The specified table of MS-health thresholds may contain the MS-health thresholds described above for Figures 1 and 2 and may be used in determining the MS-health state. A threshold-table data structure, comprising a plurality of sets of one or more key MS-values and a plurality of MS-health threshold tables, may be stored in the data storage 442. The threshold-table data structure is operable to: (i) store the plurality of sets of key MS-values and the plurality of tables of MS-health thresholds, (ii) associate one set of key MS-values with one table of MS-health thresholds, (iii) responsive to the presentation of a set of key MS-values that is stored in the threshold-table data structure, return the associated MS-health threshold table, and (iv) responsive to the presentation of a set of key MS-values that is not stored in the threshold-table data structure, return an error indication. The threshold-table data structure may be implemented as a table (e.g., a hash table or a lookup table), relational database, a linked list, a tree structure, a trie, and/or an other data structure operable to perform the operations of the threshold-table data structure described herein.

To determine the specified table of MS-health thresholds from the plurality of MS-health threshold tables, the visual MS-health indicator 453 may display one or more messages requesting user input to specify one or more key MS-values. Responsive to the one or more messages, the user of the MS-alert system 400 may provide one or more inputs via the input device 456 to specify one or more key MS-values, such as the type of engine and/or the type of fluid, such as a type of oil used in the engine 420.

The input one or more key MS-values may be presented as a set of key MS-values to the threshold-table data structure. Responsive to finding the set of key MS-values in the threshold-table data structure, the threshold-table data structure may return a table of MS-health thresholds associated with the set of key MS-values, the returned table may be determined to be the specified table of MS-health thresholds, and a message confirming the determination of the specified table of MS-health thresholds may be displayed on the visual MS-health indicator 453. If the set of key MS-values is not found in the threshold-table data structure, the threshold-table data structure may return an error indication and an error message may be displayed on the visual MS-health indicator 453 to indicate that a threshold table was not found.

Figure 6C depicts an exemplary embodiment of user interface 450, wherein the visual MS-health indicator 453 comprises a stop-light display 510 and the input device 456 comprises a keypad. Figure 6C depicts an indication of the critical MS-health state by showing the critical graphical indicator 520 as shaded and the critical textual indicator 525 as written in a bold and underlined typeface.

The MS-alert system 400 may inform a user of the MS-alert system 400 before altering one or more engine-performance characteristics. Figure 6C shows a critical alert message 570 to inform the user that the MS-alert system 400 will alter one or more MS-performance characteristics and shut down an MS (e.g., the engine 420) in five seconds. The user may be able to provide input to override an action of the MS-alert system 400, as indicated by the "Press any key to override" portion of the critical alert message 570. If the user provides input by pressing any key on the keypad within the five seconds, the MS-alert system 400 will not shut down the MS.

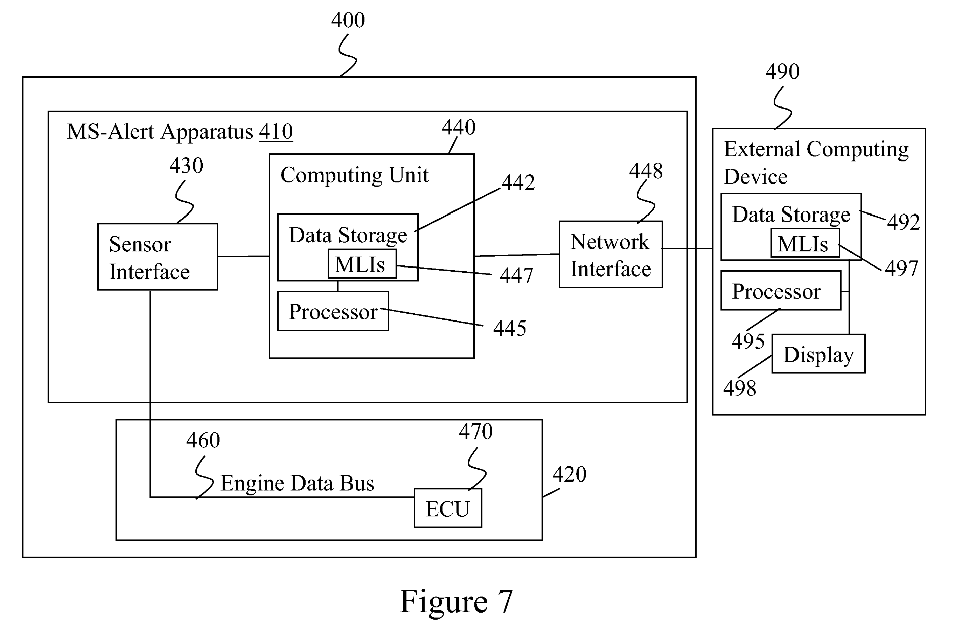

Figure 7 is a block diagram of the MS-alert apparatus 410 of the MS-alert system 400 connected to an external computing device 490 via a network interface 448. As shown in Figure 7, the MS connected to MS-alert apparatus 410 is the engine 420. The network interface 448 may interface to a wired and/or wireless connection between the MS-alert apparatus 410 and the external computing device 490.

The external computing device 490 may be any computing device, stationary or portable, with data storage 492 and a processor 495. The data storage 492 stores the MLIs 497. The processor 495 executes the MLIs 497. The MLIs 497 are machine instructions that enable the processor 495 to perform some or all of the functions of the external computing device 490 described herein. The external computing device 490 may comprise a display 498. The display 498 is operable to visually display text and graphical information to a user of the external computing device 490. The display 498 may be any device known or to be developed operable to visually display text and/or graphical information, such as a cathode ray tube and/or a liquid crystal display.

Once connected to the MS-alert apparatus 410, the external computing device 490 may obtain data from and/or download data to other components of the MS-alert system 400, including but not limited to, obtaining data from the computing unit 440 and/or the sensor interface 430 and downloading data to the computing unit 440 and/or the ECU 470. Specifically, the external computing device 490 may obtain MS-health records and/or other data stored in the data storage 442. The external computing device 490 may obtain MS-measurement data from the sensor interface 430. The external computing device 490 may download data to the computing unit 440, such as a plurality of MS-health threshold tables or MLIs 447. The data downloaded to the computing unit 440 may be stored in the data storage 442. Also, the external computing device 490 may send engine-control requests to the ECU 470 and/or receive engine-control responses from the ECU 470 via the network interface 448, the computing unit 440, the sensor interface 430, and the engine data bus 460.

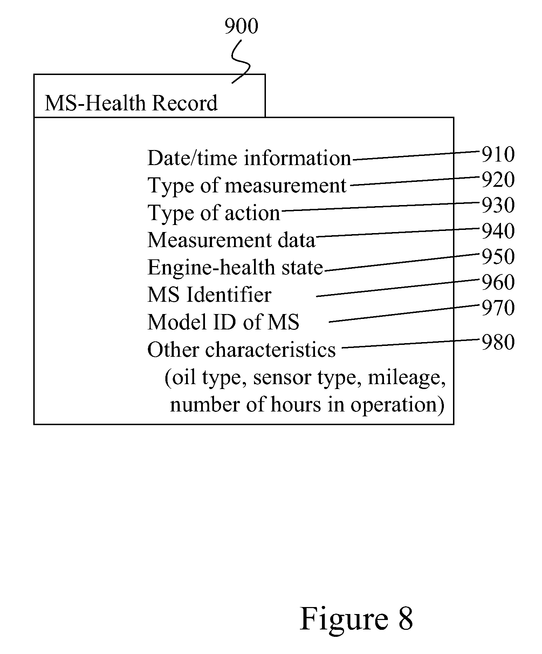

Figure 8 shows an exemplary format for a MS-health record 900. A MS-health record 900 stores MS-health data about a measurement or action taken in the MS-alert system 400. A MS-health record 900 may be stored in one or more formats including, but not limited to, as a row in a table, as part or all of a custom data structure, as part or all of a row in a relational database table and/or as part or all of a flat file. The MS-health records for each MS of a plurality of MSs, such as a plurality of engines in a fleet of motor vehicles, may be aggregated in the data storage 442 or the external computing device 490. The aggregated MS-health records may be processed to determine trends in the plurality of MSs and to generate a fleet-wide health report for the plurality of MSs.

As indicated in Figure 8, a MS-health record 900 may have a plurality of fields storing MS-health data, including, but not limited to, a date/time information field 910, a type of measurement field 920, a type of action field 930, a measurement data field 940, and a MS-health state field 950. The type of measurement field 920 may indicate a type of measurement taken, such as, but not limited to, a MS-health measurement, an oil-viscosity measurement, a fuel-flow measurement, or an air-flow measurement. The type of action field 930 may record an action was taken and/or an effect of an action taken by the MS-alert system 400, by specifying a type of action in the type of action field 930. Examples of the type of action are engine-control commands and engine-control requests. The measurement data field 940 stores the value and/or other information about the measurement or action being recorded in the MS-health record, such as a value of "50" of a MS-health measurement. The MS-health state field 950 stores a value of the MS-health state of the MS, such as "normal."

A MS-health record 900 may store a plurality of engine characteristic fields, such as an MS identifier field 960, a model identifier of the engine field 970, and an other characteristics field 980. The MS identifier field 960 may record an identifier for a specific MS, such a Vehicle Identification Number (VIN) for a vehicle, serial number for an engine or other MS, or similar identifiers. The model identifier of the MS field 970 may specify a type of MS, such as a "CarMaker 531C" vehicle engine. The other characteristics field 980 may store information about a MS, including, but not limited to, the type of fluids used, a brand or other type of oil or other fluid used, the types of sensor(s) used to determine the MS-health measurement, the mileage and/or the operation time of the MS.

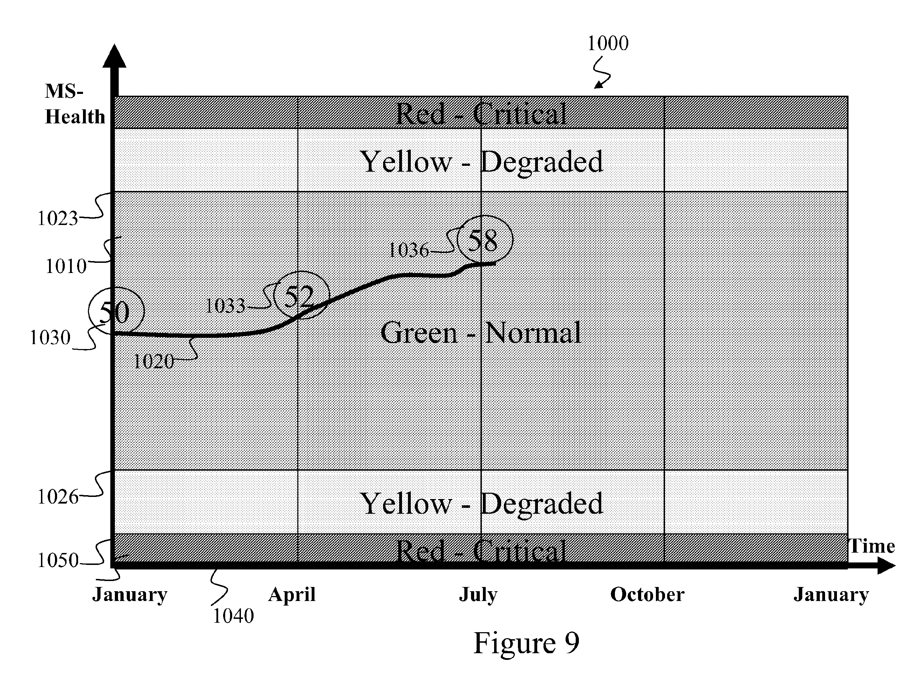

Figure 9 is a trend analysis diagram 1000 for an engine, indicating both a MS-health state 1010 and a MS-health measurement graph 1020. Figure 9 shows the MS-health state 1010 as "normal" in trend analysis diagram 1000 as the MS-health measurement graph 1020 is between normal thresholds 1023 and 1026 during the time span shown in the trend analysis diagram 1000. The trend analysis diagram 1000 may be based, at least in part, by user input, such as input specifying the time span. Figure 9 shows the trend analysis diagram 1000 plotted on a time axis 1040 and a MS-health measurement axis 1050. While the time axis 1040 is shown as quarterly in Figure 9, it is to be understood that the trend analysis diagram 1000 may indicate shorter or longer time scales.

A trend analysis diagram 1000 may depict the MS-health measurement graphically and/or textually. Figure 9 shows the trend analysis diagram 1000 with the MS-health measurement graph 1020 and textual MS-health measurements 1030, 1033, and 1036. The textual MS-health measurements may indicate specific values of the MS-health measurement graph 1020 at specific times. Figure 9 shows the textual MS-health measurements 1030, 1033, and 1036, indicating the respective specific values of 50 in January, 52 in April, and 58 in July.

A trend analysis diagram 1000 may be generated based, at least in part, on information stored in a plurality of MS-health records, each of which may be a MS-health record 900. Based on the value of the type of measurement field 920 of a MS-health record 900, the computing unit 440 may determine that a MS-health record 900 in the plurality of MS-health records corresponds to a MS-health measurement. A MS-health record 900 corresponds to a MS-health measurement if a value of the type of measurement field 920 of the MS-health record 900 indicates that the value of the type of measurement field 920 is that of a MS-health measurement.

Responsive to the determination, each MS-health record in the plurality of MS-health records that corresponds to a MS-health measurement may be sorted in chronological order by sorting the plurality of MS-health records based on a value of the date/time information field 910 of each MS-health record 900. If a time span is specified for a specific trend analysis diagram, a MS-health record 900 in the plurality of MS-health records may be used to generate the specific trend analysis diagram if the value of the date/time information field 910 is within the specified time span. For each MS-health record 900 in the sorted plurality of MS-health records, the trend analysis diagram 1000 may be generated by plotting the point (x,y), where x is the date/time information field value 910 value of the MS-health record, and y is the value of the measurement data field 940 of the MS-health record 900. The point (x,y) may be plotted graphically using a mark (e.g. a dot), as part of a line graph, and/or as a bar in a bar graph. The trend analysis diagram 1000 may provide a textual or numerical representation of the point (x,y).

While not shown in Figure 9, a trend analysis diagram 1000 may comprise an MS-measurement data graph, such as an oil-viscosity measurement graph. The oil-viscosity MS-measurement data graph may be generated from a plurality of MS-health records in a similar fashion as described above for generating a trend analysis diagram, by replacing the determination that a value of type of measurement field 910 indicates a MS-health measurement with a determination that the value of the type of measurement field 910 indicates an oil-viscosity measurement. The other MS-measurement data may be shown as a graph with time on the horizontal axis and the other MS-measurement data on the vertical axis.

The computing unit 440 may generate the trend analysis diagram 1000 and display the trend analysis diagram 1000 on the user interface 450. Alternatively, the external computing device 490 may generate the trend analysis diagram 1000 and/or display the trend analysis diagram 1000 on the display 498 of the external computing device 490. For example, a plurality of MS-health records 900 may be made available to the external computing device 490 operable as a planning tool, such as the exemplary convoy planning tool described in U.S. Patent Application Number 11/955,198. The planning tool may generate the trend analysis diagram 1000, based, at least in part, on data stored in the plurality of MS-health records 900. The trend analysis diagram 1000 may be generated with custom software, general database software written by vendors such as Honeywell International, Inc. of Morristown, N.J., internal database tools, or by a plurality of software resources.

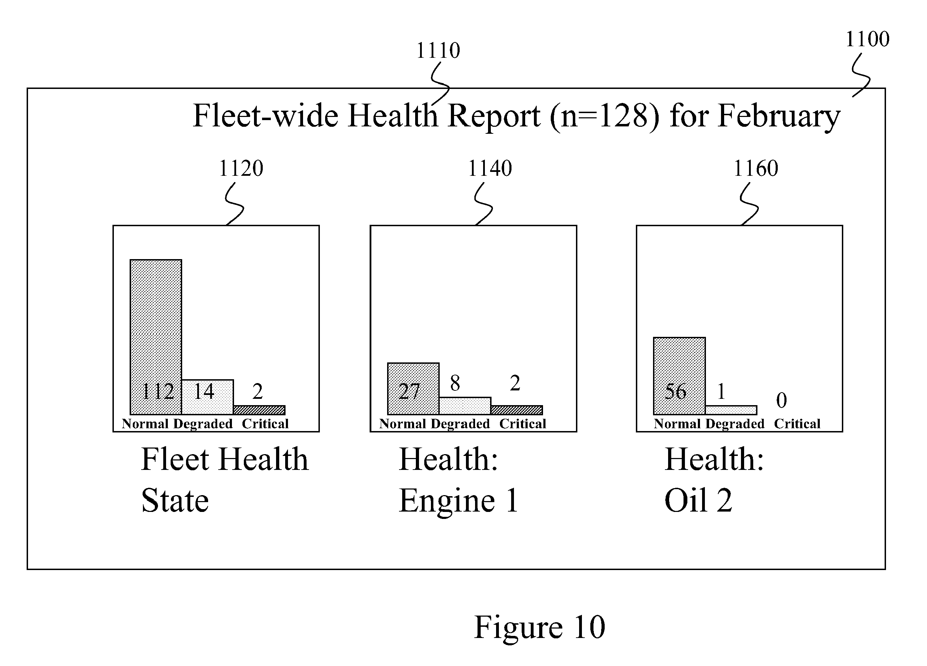

Figure 10 shows a fleet-health diagram 1100 with a title 1110 and three fleet-health sub-diagrams 1120, 1140, and 1160 for a fleet of 128 vehicles (i.e., the vehicles are MSs) during the month of February. The number of vehicles in the fleet is indicated by the "n=128" portion of the title 1110. Figure 10 also shows the fleet-health state sub-diagram 1120 indicating MS-health state over a period of time (the month of February) for the fleet of vehicles, the fleet-health state sub-diagram 1140 indicating MS-health state over a period of time based on a particular model of engine used in the fleet of vehicles, and the fleet-health state sub-diagram 1160 indicating MS-health state over a period of time based on a particular type of oil used in the engines of the fleet of vehicles. While fleet-health diagram 1100 is shown with bar graphs in Figure 10, it is to be understood that fleet-health diagram may be shown as a pie graph, line graph, and/or other types of graphs. The trend analysis diagram 1000 and/or the fleet health report 1100 may be made available to users on paper, by fax, electronically, and/or by other data communication means.

The fleet-health report 1100 may include a fleet-health state. Figure 10 shows the fleet-health state sub-diagram 1120 of the fleet-health report 1100 as indicating 112 vehicles with a normal MS-health state, 14 vehicles with a degraded MS-health state and 2 vehicles with a critical MS-health state.

The fleet-health state may be a worst MS-health state. The worst MS-health state of an engine may be determined during a time period as indicated in MS-health records for a particular engine of a vehicle in the fleet of vehicles. A worst MS-health state may depend on a classification of MS-health states. For example, if there are three MS-health states of "normal", "degraded", and "critical", the critical state may be classified as worse than the degraded state, and the degraded state may be classified as worse than the normal state.

Using the example classification, an exemplary determination of the worst MS-health state for the particular engine during a time period is: (1) if there is a MS-health record with a critical MS-health state for the particular engine during the time period, the worst MS-health state is critical, otherwise (2) if there is a MS-health record with a degraded MS-health state for the particular engine during the time period, the worst MS-health state is degraded, otherwise (3) the worst MS-health state is normal.

For an example of the exemplary determination, if an engine starts a time period with a MS-health record indicating a "normal" MS-health state and then a later MS-health record, as recorded during the time period, indicates a "degraded" MS-health state, the worst MS-health state of the engine in that time period is determined to be "degraded." As another example, if an engine starts a time period with a MS-health record indicating a "critical" MS-health state and then a later MS-health record, as recorded during the time period, indicates a "degraded" or "normal" MS-health state, the worst MS-health state of the engine in that time period is determined to be "critical."

The fleet-health report 1100 may correspond to a particular engine type used in each vehicle in the fleet of vehicles. As shown in Figure 10, the fleet-health sub-diagram 1140 indicates 27 engines with a particular engine type of "Engine 1" have a worst MS-health state of normal, 8 engines with the particular engine type of "Engine 1" have a worst MS-health state of degraded, and 2 engines with the particular engine type of "Engine 1" have a worst MS-health state of critical. Preferably, the particular engine type is determined from data stored in a plurality of MS-health records 900.

The fleet-health report 1100 may correspond to a particular type of oil used in each vehicle in the fleet of vehicles. As shown in Figure 10, the fleet-health sub-diagram 1160 indicates 56 engines with a particular type of oil of "Oil 2" have a worst MS-health state of normal, 1 engine with the particular type of oil of "Oil 2" has a worst MS-health state of degraded, and no engines with the particular type of oil of "Oil 2" have a worst MS-health state of critical. Preferably, the particular type of oil is determined from data stored in the plurality of MS-health records 900.

### 4. Method for Indicating a MS-health State

Figure 11 is a flowchart describing a method 1200 for indicating a MS-health state of an engine 420. At block 1210, MS-measurement data are obtained. MS-measurement data are data about one or more MS-measurements of an MS (e.g. the engine 420), including fluid-measurement data, (e.g., oil-viscosity measurement data of engine oil in the engine 420). The MS-measurement data may be obtained from (1) one or more MS-sensors and/or (2) data transmissions on an data bus, such as the engine data bus 460.

At block 1220, the MS-measurement data are transmitted to a computing unit 440. The computing unit 440 may receive the transmitted MS-measurement data using a sensor interface 430 that is wired and/or wireless.

At block 1230, the computing unit 440 calculates a MS-health measurement of the MS based, at least in part, on the transmitted MS-measurement data. To calculate the MS-health measurement, the computing unit 440 may use the fluid-measurement data, such as oil viscosity measurement data, as the MS-health measurement, perform a simple or weighted average of the oil viscosity measurement data over a period of time, and/or otherwise calculate the MS-health measurement using the transmitted MS-measurement data.

A plurality of MS-health measurements may be stored in data storage 442 and/or on an external computing device 490. The computing unit 440 may determine the MS-health measurement based, at least in part, on the stored plurality of MS-health measurements. In particular, the computing unit 440 may determine the MS-health measurement as an average of a current MS-health measurement and one or more MS-health measurements in the stored plurality of MS-health measurements. The current MS-health measurement may be stored along with the MS-health measurements used in calculating the current MS-health measurement.

If the MS-measurement data comprises measurements from more than one type of sensor, an average of the measurements in the MS-measurement data may be calculated to determine the MS-health measurement. The determined average may be a simple average or a weighted average of the measurements. The measurements in the MS-measurement data may be scaled to a common range of reference values before averaging the measurements.

For example, suppose the MS-health measurement was determined using measurements from an oil-viscosity sensor 434 and a fuel-flow sensor. Suppose the oil-viscosity sensor 434 provides oil-viscosity measurements using values in the range of [0,50] and the fuel-flow sensor provides fuel-flow measurements using values in the range of [0,20]. At a given time, further suppose the oil viscosity sensor 434 provides a value of 20 and the fuel-flow sensor provides a value of 10. A simple average of the two values would be 15.

Suppose that both values are to be scaled to a [0,100] scale. As the desired scale comprises 100 units, and the oil-viscosity sensor 434 provides values with a range of 50 units, the oil-viscosity values are scaled by multiplying the oil-viscosity values by 100/50 = 2. Similarly, to scale the fuel-flow values to a [0,100] scale, the fuel-flow values are multiplied by 5. For the example given, the scaled oil-viscosity value is 40 and the scaled fuel-flow value is 50. A simple average of the scaled values would be 45. Suppose further that the MS-health measurement is calculated using a weighted average of 90% of the scaled oil-viscosity value + 10% of the scaled fuel-flow value. For the example scaled values and weights, the weighted average and, therefore, the MS-health measurement would be 90%*40+10%*50 = 41.

At block 1240, the computing unit 440 determines the MS-health state of the MS based, at least in part, on the MS-health measurement. Preferably, the MS-health state is in one of three possible states: normal, degraded, and critical. However, the MS-health state may be in a state other than normal, degraded, or critical.

The computing unit 440 may determine the MS-health state by comparing the MS-health measurement with one or more MS-health thresholds. The one or more MS-health thresholds may be stored by the computing unit 440 in the data storage 442. The one or more MS-health thresholds may be stored in a data structure operable to store the one or more MS-health thresholds and later allow the one or more MS-health thresholds to be retrieved, such as a specified table of MS-health thresholds.

The specified table of MS-health thresholds may be determined by presenting a set of key MS-values to a threshold-table data structure. The threshold-table data structure may return a table of MS-health thresholds, which may be determined to be the specified table of MS-health thresholds. One or more key MS-values in the set of key MS-values may be determined based on user input to the MS-alert system 400.

### Table 1 below is an example of a specified table of MS-health thresholds:

**Table 1**

| **Threshold** | **Value** |
|---|---|
| Upper Degraded Threshold | 90 |
| Upper Normal Threshold | 80 |
| Lower Normal Threshold | 20 |
| Lower Degraded Threshold | 10 |

In this example, the MS-health measurement corresponds to a critical MS-health state if the MS-health measurement is greater than the upper degraded threshold or less than the lower degraded threshold. Using the values of Table 1, if the MS-health measurement is greater than 90, the upper degraded threshold, the MS-health state is determined to be critical, as the MS-health measurement is above the upper degraded threshold. Similarly, the MS-health state is determined to be critical if the MS-health measurement is less than 10, the lower degraded threshold.

Also, the MS-health measurement corresponds to a degraded MS-health state if the MS-health measurement is between a normal threshold and the corresponding degraded threshold. Using the values of Table 1, if the MS-health measurement is between 80, the upper normal threshold, and 90, the upper degraded threshold, the MS-health state is determined to be degraded, as the MS-health measurement is between the upper normal threshold and the upper degraded threshold. Similarly, the MS-health state is determined to be degraded if the MS-health measurement is between 10, the lower degraded threshold, and 20, the lower normal threshold.

Further, the MS-health measurement corresponds to a normal MS-health state if the MS-health measurement is between the normal thresholds. Using the values of Table 1, if the MS-health measurement is between 20, the lower normal threshold, and 80, the upper normal threshold, the MS-health state is determined to be normal. It is to be understood that more or fewer MS-health states can be used by adding more or fewer thresholds, respectively, and performing similar comparisons of the MS-health measurement to the MS-health thresholds.

If the MS-health measurement is the exact value of a threshold, the MS-health state may be in either of the two MS-health states separated by the threshold. A threshold separates two MS-health states S1 and S2, where S1 is the MS-health state when the MS-health measurement is below the threshold and S2 is the MS-health state when the MS-health measurement exceeds the threshold. If the MS-health measurement is the exact value of a threshold, the MS-health state may be determined to be the worst MS-health state of the two states separated by the threshold, the previous MS-health state (if that state was one of the two states separated by the threshold), or a random or other choice between the two states separated by the threshold.

For example, if a MS-health measurement is 80, then the MS-health measurement is the exact value of the upper normal threshold of Table 1. The upper normal threshold separates the normal MS-health state and the degraded MS-health state. For a MS-health measurement of 80, the MS-health state may be either normal or degraded. If the worst MS-health state is used when the MS-health measurement is the exact value of the threshold, then based on the worst MS-health state classification described above, the MS-health state for a MS-health measurement of 80 would be "degraded."

Preferably, the values of the MS-health thresholds are determined before operating the MS-alert system 400. The value of each MS-health threshold may be determined by operating or otherwise testing a test MS, such as an engine, to determine a time where the MS-health state changes for the test engine. At the time where the MS-health state changes for the test MS, MS-measurement data for the test MS is obtained. Preferably using the same determination used by the MS-alert system 400 to determine the MS-health measurement value, the value of the MS-health threshold is determined based, at least in part, on the obtained MS-measurement data for the test MS. For example, if a MS-health measurement value is determined using the weighted average of the oil-viscosity sensor and the fuel-flow sensor measurements described in the example above, preferably the same weighted average is used to determine the values of the MS-health thresholds.

At block 1250, the computing unit 440 determines whether to store MS-health records 900. If the MS-health records 900 are not to be stored, the method 1200 proceeds to block 1260; otherwise, the method 1200 proceeds to block 1255. At block 1255, the computing unit 440 creates and stores the MS-health record 900, preferably in the data storage 442. The external computing device 490 may retrieve one or more MS-health records 900. Based, at least in part on information in the one or more MS-health records 900, the external computing device 900 may determine a MS-health measurement value or generate graphs, such as a trend analysis graph 1000, a fleet-health report 1100, or an MS-measurement data graph.

At block 1260, the MS-health state of the MS, such as the engine 420, is indicated. The MS-health state of the MS may be indicated visually, aurally or using a combination of visual and aural indications. A stop-light indicator may provide the visual indication of the MS-health state.

At block 1270, computing unit 440 determines whether the MS-health state has changed from a previous MS-health state. If the MS-health state has not changed, the method 1200 proceeds to block 1210. However, if the MS-health state has changed, the method 1200 proceeds to block 1275 where one or more MS-performance characteristics, such as engine-performance characteristics, may be altered. For example, if the MS-health state has changed from normal to degraded, the MS-performance characteristic of fuel flow may be reduced, and if the MS-health state has changed from either normal or degraded to critical, the fuel flow may be stopped. In another example, if the MS-health state has changed from either critical or degraded to normal, the fuel flow may be increased. Further, if the MS-health state is changed, the previous MS-health state is updated to be the MS-health state. After executing block 1275, the method 1200 proceeds to block 1210.

### 5. Conclusion

It should be understood, however, that this and other arrangements described in detail herein are provided for purposes of example only and that the invention encompasses all modifications and enhancements within the scope and spirit of the following claims. As such, those skilled in the art will appreciate that other arrangements and other elements (e.g. machines, interfaces, functions, orders, and groupings of functions, etc.) can be used instead, and some elements may be omitted altogether.

Further, many of the elements described herein are functional entities that may be implemented as discrete or distributed components or in conjunction with other components, in any suitable combination and location, and as any suitable combination of hardware, firmware, and/or software. A CU such as ECU 470 and/or the external computing unit 490 may carry out one or more of the functions described herein as being carried out at the computing unit 440. Similarly, the computing unit 440 and/or the external computing unit 490 may carry out one or more of the functions described herein as being carried out at a CU such as the ECU 470. Further and similarly, the computing unit 440 and/or a CU such as the ECU 470 may carry out one or more of the functions described herein as being carried out at the external computing unit 490.

## Claims

1. An apparatus for indicating a mechanical-system-(MS)-health state of a MS comprising:
a user interface;
a sensor interface for receiving MS-measurement data, wherein the MS-measurement data comprises at least one fluid-viscosity measurement value;
a processor;
data storage; and
machine language instructions stored in the data storage and executable by the processor to perform functions comprising:
determining a MS-health state of the MS, based, at least in part, on the received MS-measurement data, and
indicating the MS-health state via the user interface.

2. The apparatus of claim 1, wherein the MS is an engine and the fluid-viscosity measurement value is a oil-viscosity measurement value.

3. The apparatus of claim 1, wherein the functions further comprise:
determining a MS-health measurement value based, at least in part, on the received MS-measurement data; and
comparing the determined MS-health measurement value to one or more MS-health thresholds, so as to determine the MS-health state.

4. The apparatus of claim 3, wherein the MS-health measurement value is the fluid-viscosity measurement value.

5. The apparatus of claim 3, wherein the one or more MS-health thresholds comprise at least two thresholds, so as to determine that the MS-health state is in one of at least three possible MS-health states.

6. The apparatus of claim 3, wherein the user interface comprises:
a MS-health indicator operable to indicate the MS-health state; and
an input device.

7. The apparatus of claim 6, wherein the functions further comprise: receiving at least one key value from the input device, and determine at least one MS-health threshold based, at least in part, on the received at least one key value.

8. The apparatus of claim 3, wherein determining a MS-health measurement value comprises:
storing a plurality of MS-health measurement values in the data storage; and
determining the MS-health measurement value based, at least in part, on an average of the stored plurality of MS-health measurement values.

9. The apparatus of claim 3, wherein the MS-measurement data comprises data from at least two MS sensors, and
wherein the functions further comprise processing the data from the at least two MS sensors, so as to determine a MS-health measurement value.

10. The apparatus of claim 2, further comprising a network interface, wherein the network interface is communicatively coupled to the processor and a second processor,
wherein the second processor comprises a convoy planning tool,
whereby the processor sends at least one MS-health record to the second processor,
wherein the at least one MS-health record comprises the determined MS-health measurement value, a time of the determined MS-health measurement value, a MS identifier, and a model identifier of the MS, and
wherein the convoy planning tool of the second processor generates at least one diagram selected from the group consisting of a trend analysis diagram and a fleet-health diagram.
